# EUROPEAN PATENT SPECIFICATION

(11) **EP 2 389 805 B1**
(45) Date of publication and mention of the grant of the patent: **12.02.2014**
(21) Application number: 11167709.2
(22) Date of filing: 26.05.2011
(51) Int. Cl.: A01N 25/00, A01N 25/10, A01N 63/00, A01K 67/033, A01P 9/00

(54) **Biological Control of Molluscs with nematodes**
Biologische Kontrolle von Mollusken mit Nematoden
Contrôle biologique de mollusques avec nématodes

(30) Priority: 27.05.2010 GB 201008877
(43) Date of publication of application: 30.11.2011
(73) Proprietor: BASF Agricultural Specialities Limited, Littlehampton, Sussex BN17 7AU (GB)
(72) Inventor: Pearce, Jeremy, David, Littlehampton, Sussex BN17 7AU (GB); Matcham, Stephen, Edward, John, Littlehampton, Sussex BN17 7AU (GB); Morgan, Robert, Leonard, Littlehampton, Sussex BN17 7AU (GB); Brown, Andrew, Peter, Littlehampton, Sussex BN17 7AU (GB)
(74) Representative: Jabs, Thorsten

(56) References cited:
- WO-A1-2009/050482
- NL-C2- 1 033 726
- US-A- 5 401 506
- US-A1- 2005 281 854
- MICHAEL J. WILSON ET AL: "Monoxenic culture of the slug parasite Phasmarhabditis hermaphrodita (Nematoda : Rhabditidae) with different bacteria in liquid and solid phase", FUNDAM. APPL. NEMATOL.,, vol. 18, no. 2, 1 February 1995 (1995-02-01), pages 159-166, XP55040268,

## Description

### Field of the invention

The present invention relates to the control of agricultural and horticultural pests and more particularly to the control of molluscs, including slugs, e.g. *Deroceras reticulatum* and snails, e.g. *Monacha cantiana.*

### Background of the invention

Slugs are a widespread pest of several major agricultural crops, particularly winter wheat, oilseed rape and potatoes. They are also a problem in horticulture and to the domestic gardener. The most economically important slug species is the grey field slug, *Deroceras reticulatum* (family: Limacidae), although other limacid slugs and *Arion* (family: Arionidae), *Tandonia, Milax* (family: Milacidae) and *Boettgerilla* species also can cause significant damage. Snails also can be a pest problem in horticulture and agriculture, one example being *Monacha cantiana* (family: Heilcidae). Examples of mollusc pests are listed by Godan in "Pest Slugs and Snails" (1983, Springer-Verlag, Berlin). Molluscs may also carry pests which represent a hazard to human or animal health. Examples include *Lymnaea* species (family: Lymnaeidae), which carry the liver fluke *Fasciola hepatica,* and *Bulinus* species (family: Bulinidae) which carry *Opisthorcis sinensis.* The families Limacidae, Arionidae, Milacidae and Helicidae are members of the Order Stylommatophora. The families Bulinidae and Lymnaeidae are members of the Order Basommatophora.

Conventional methods of controlling slugs are generally based on the toxins methiocarb or metaldehyde, which can be applied as liquids, seed treatments or baits. Molluscicides are different from most pesticides in that they are commonly applied in a bait form (Bourne et. al. 1988, Slug feeding behavior in relation to control with molluscicidal baits. J Moll Stud 54, 327-338; Henderson and Triebskorn Chemical control of terrestrial gastropods In Barker GM (Ed) Molluscs as Crop pests CABI publishing pg 1-32). More recently baits have become available based on chelated iron phosphate (Edwards et. al. 2009 The relative toxicity of metaldehyde and iron phosphate-based molluscicides to earthworms. Crop protection 29, 289-294).

Nematodes of the genus *Phasmarhabditis* are parasites of a wide range of mollusc species, in particular slugs and snails, and so are effective control agents for these species. Particularly effective *Phasmarhabditis* species are the related organisms *Phasmarhabditis neopapillosa* and *Phasmarhabditis hermaphrodita.* These species have been known for many years and are described in the literature, having been characterised especially by Andrassy in "A Taxonomic Review of the Sub-Order Rhabditina (Nematoda:Secernentina)" (1983, Orstom, Paris). Their use in the biological control of slugs is described in WO 93/00816.

*P. hermaphrodita* works in a different manner to slug baits in that the nematodes are generally applied to the soil, where they can actively seek out, infect and kill slugs. *P*. *hermaphrodita* is sold commercially as a biological control agent for slugs into the domestic, horticultural and agricultural markets. This product is called Nemaslug^{RTM} by Becker Underwood. Nemaslug^{RTM} contains nematodes in their vigorously infective stage. The nematode infects the slug by entering the slug's mantle, where they reproduce and cause the mantle to swell. The slug dies and the nematodes reproduce further on the cadaver. When the nutrients are exhausted the nematodes form dauer infective stage nematodes which then leave and search for other molluscs. The main drawback of this nematode as a control agent is its high price in relationship to existing chemical alternatives. The present invention can overcome this problem.

GB 24663501 describes a composition based on application of *P.hermaphrodite* through a rain gun composition. The rain gun provides a different mechanism by which the nematodes are delivered to the target.

A recent development of using nematodes to control slugs has been to combine *P*. *hermaphrodita* with a potato bait for the slugs. Using this method the slugs are attracted to the nematode-laden potato bait, which they eat and so become infected by the nematodes. This composition is analogous to conventional slug pellets, except that it contains an infective biological control agent. This product is called Nematode SPS and is supplied by Horticoop, Klappolder 150, 2665 LP Bleiswijk, Netherlands.

NL1033726 discloses a mollusc bait which uses *P. hermaphrodita* nematodes loaded onto a solid carrier comprising cooked potato.

US5401506 discloses an insecticidal composition comprising a first composition having an effective amount of at least one species of entomopathogenic nematode distributed in a matrix, surrounded by a second composition selected from vegetable oil, crop oil and partially hydrogenated oil containing mono- and di-glycerides.

WO2009/050482 discloses pesticidal compositions comprising a biopesticide, an absorbent and water.

Wilson et al. (Fundam. Appl. Nematol., 18(2) 159-166) relates to the monoxenic culture of *P. hermaphrodita* with different bacteria in liquid and solid phase.

US2005/281854 relates to novel materials for controlling molluscs using carbohydrates including celluloses, hemi-cellulose complexes, and/or lignin, for inducing death in molluses.

The current invention takes current methods of controlling slugs a stage further.

### Summary of the invention

According to a first aspect the invention provides a composition for controlling molluscs comprising a mollusc bait and a nematode species of the genus *Phasmarhabditis*, wherein the bait comprises a superabsorbent polymer, a nitrogen source and water. The bait is designed to allow growth and reproduction of the infective agent, e.g. *P. hermaphrodita*. Molluscs, such as slugs, are attracted to the bait which also acts as a food source for the infective agent. This increases the number of infective agents able to kill molluscs. This is useful as it increases the level of control and increases the efficacy of the nematodes applied via an amplification effect of the infective agent.

According to a second aspect the invention provides a kit for controlling molluscs which comprises (i) a mollusc bait and (ii) *Phasmarhabditis* nematodes, wherein the mollusc bait comprises a superabsorbent polymer and a selected nitrogen source. The bait, when hydrated, provides an environment suitable for reproduction of the nematode.

The nitrogen source is selected from one or more of brans of rice, corn, wheat, oats and barley; chicken layers mash; whole yeast; yeast extracts; yeast peptones; powdered whole egg; egg yolk; casein; casein peptones; extracts and peptones of meats, fish, dairy products; protein extracts and peptones of plant matter e.g. soya protein, soya isolates and soya peptone; and animal feeds of dairy cows, beef cows, sheep, pigs or poultry.

Preferably the nematode is *Phasmarhabditis neopapillosa* or *Phasmarhabditis hermaphrodita,* most preferably *Phasmarhabditis hermaphrodita.* Preferably the nematode is present as dauer larvae.

Preferably the mollusc bait further comprises an emulsified vegetable lipid component. Preferably the mollusc bait comprises from 2 to 50 % by weight of a nitrogen source, based on the weight of the hydrated bait. Preferably the mollusc bait comprises from 0.25 to 10 % by weight of a superabsorbent polymer, based on the weight of the hydrated bait. Preferably the mollusc bait comprises from 0.25 to 20 % by weight of a lipid component, based on the weight of the hydrated bait. Preferably the mollusc bait comprises from 1 to 5000 infective juvenile nematodes per gram of hydrated bait.

According to another aspect the invention provides a method of making a composition for controlling molluscs comprising providing a composition comprising a superabsorbent polymer and a selected nitrogen source, adding water to the composition to form a hydrated mollusc bait, and adding a nematode species of the genus *Phasmarhabditis.*

According to a further aspect the invention provides a method of controlling molluscs which comprises applying to an area subject to infestation therewith a composition as described herein. According to yet a further aspect the invention provides a method of controlling molluscs which comprises taking a kit as described herein, adding water to the mollusc bait therein, adding the nematodes to the hydrated mollusc bait and applying the resulting composition to an area subject to infestation with molluscs.

### Brief description of the drawings

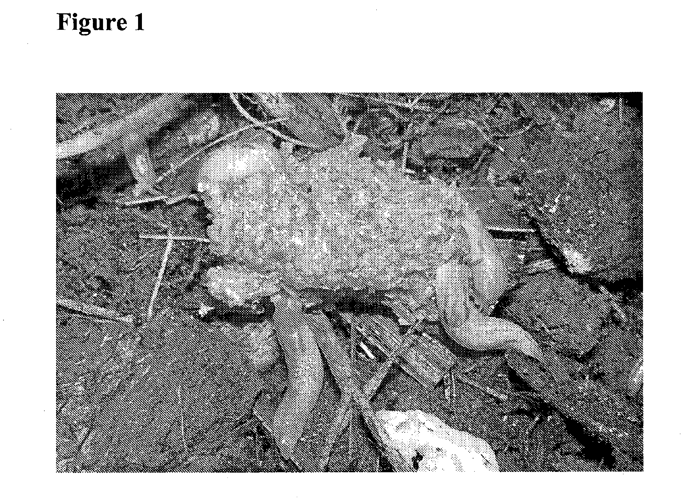

Fig. 1 shows slugs feeding on a molluscicidal bait composition according to the invention.

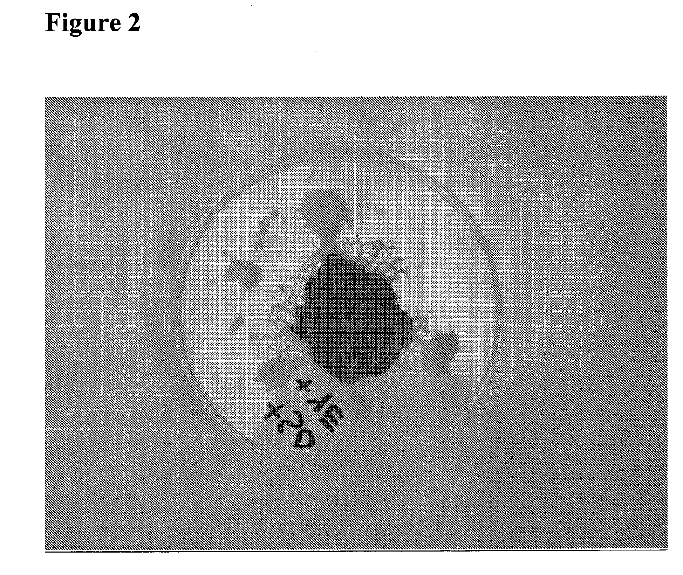

Fig. 2 shows nematodes migrating from a molluscicidal bait composition according to the invention after having reproduced.
Fig. 3 shows number of *P. hermaphrodita* vs time with 3 different baits (control - diamond, bran gel - square, and potato - triangle).
Fig. 4 shows an undamaged Cymbidium step (top) and a slug-damaged flower (bottom).
Fig. 5 shows percent cumulative damage of Cymbidium sp. (variety Malinga) stems treated with different slug control regimes vs time.
Fig. 6 shows percent cumulative damage of Cymbidium sp. (variety Evergreen) stems treated with different slug control regimes vs time.
Fig. 7 shows number of *P. hermaphrodita* vs time with 3 different baits (bran gel - diamond, bran gel plus lipid - square, and bran gel plus nitrogen - triangle).

### Detailed description of the invention

The molluscicide of the invention is based on the combination of a bait with an infective control agent, such as *P. hermaphrodita,* to promote growth and reproduction of the infective agent. This combination is effective for controlling molluscs. The term "controlling molluscs" is to be understood to encompass warding off, deterring or killing mollusc pests, such as slugs and snails, which are harmful to plants in the garden, field or greenhouse, or which carry parasites harmful to humans or animals, as discussed above. The aim of "controlling molluscs" is to protect plants which would otherwise be damaged by molluscs and/or to prevent the spread of harmful parasites carried by molluscs.

Any nematode of the genus *Phasmarhabditis* may be used in accordance with the invention. Preferred nematodes are *Phasmarhabditis neopapillosa* and *Phasmarhabditis hermaphrodita,* particularly *Phasmarhabditis hermaphrodita.* The organisms can be obtained from slugs in the field and cultured by methods described in WO 93/00816 to produce amounts sufficient for formulation into the compositions described herein for application in the field or greenhouse. Alternatively, nematodes can be purchased commercially, for example Nemaslug^{RTM} from Becker Underwood.

Preferably the nematode is initially present in the compositions of the invention as dauer larvae. According to the invention it is possible to use from 1 to 5000 infective juvenile nematodes per gram of hydrated bait. It is preferable to use from 20 to 3000 infective juvenile nematodes per gram of hydrated nutrient bait, or from 100 to 2000 infective juvenile nematodes per gram of hydrated mollusc bait, or from 1000 to 1500 infective juvenile nematodes per gram of hydrated mollusc bait. Of course, the nematodes will grow and reproduce on the mollusc bait and so with time the concentration of nematodes will increase.

The nematodes are combined with a mollusc bait which provides an environment suitable for reproduction of the nematode. In particular the bait is designed to provide nutrients to the nematodes to enable them to grow and reproduce. Thus, the bait preferably comprises a superabsorbent polymer and a nitrogen source. The bait may also comprise one or more sources of carbon and other nutrients which are useful for the growth and reproduction of *P. hermaphrodita*. In this respect, we note that although *Phasmarhabditis* are bacterial feeders, they will feed on a range of bacteria and so it is not necessary to include bacteria in the bait.

Superabsorbent polymers are polymers that can absorb and retain extremely large amounts of a liquid relative to their own mass. It will therefore act as a source of water to the nematodes in the bait. The total absorbency and swelling capacity are controlled by the type and degree of cross-linking to the polymer. Low density cross-linked superabsorbent polymers generally have a higher absorbent capacity and swell to a larger degree. These types of superabsorbent polymers also have a softer and more cohesive gel formation. High cross-link density superabsorbent polymers exhibit lower absorbent capacity and swell. The gel strength is firmer and can maintain particle shape even under modest pressure.

Preferably the superabsorbent polymer used in accordance with the invention is a copolymer of polyacrylate and/or polyacrylamide. Superabsorbent polymers are now commonly made from the polymerization of acrylic acid blended with sodium hydroxide in the presence of an initiator to form a polyacrylic acid sodium salt (sodium polyacrylate). Other materials are also used to make a superabsorbent polymer include polyacrylamide copolymer, ethylene maleic anhydride copolymer, cross-linked carboxy-methyl-cellulose, polyvinyl alcohol copolymers, cross-linked polyethylene oxide, and starch grafted copolymer of polyacrylonitrile. Most preferably the superabsorbent polymer is a cross-linked salt of sodium or potassium polyacrylate or polyacrylamide, i.e. it comprises sodium polyacrylate and/or potassium polyacrylate and/or sodium polyacrylamide and/or potassium polyacrylamide.

The nitrogen source comprises a source of nitrogen which will attract the molluscs and provide nutrients to the nematodes. For example, any nitrogen source currently used in slug baits may be appropriate. Specifically the nitrogen source is selected from one or more of brans of rice, corn, wheat, oats and barley; chicken layers mash; whole yeast; yeast extracts; yeast peptones; powdered whole egg; egg yolk; casein; casein peptones; extracts and peptones of meats, fish, dairy products; protein extracts and peptones of plant matter e.g. soya protein, soya isolates and soya peptone; and animal feeds of dairy cows, beef cows, sheep, pigs or poultry.

The mollusc bait may further comprise a lipid component, which is preferably an emulsified vegetable lipid component, to further attract the molluscs and provide additional nutrients to the nematodes. Any lipid component may be used, for example the emulsified vegetable lipid component may comprise one or more of vegetable oils such as sunflower, corn, rapeseed and soya bean oil; and emulsifiers such as lecithin, egg yolk, monoglycerides, diglycerides, sodium stearoyl acetate and sorbitan fatty acid esters.

The mollusc bait may be formulated by the skilled person to provide a composition that is both attractive to slugs and nutritious for nematodes. The bait is preferably supplied in dry form, for example as a powder or granules. Preferably, the dry bait comprises from 10 to 99 % by weight of a nitrogen source, most preferably from 80 to 95 %, or from 85 to 90 %, for example about 92 % by weight. Preferably, the dry bait contains from 1 to 40 % by weight of a superabsorbent polymer, most preferably 1 to 15 %, for example about 8 % by weight. Preferably, the dry bait contains from 1 to 80 % by weight of a lipid component, most preferably 5 to 25 %. For example about 15 % by weight. Alternatively it may be supplied as a gel.

In use the mollusc bait should be hydrated with water which will be absorbed into the superabsorbent polymer. For example, the hydrated mollusc bait may contain from 20 to 97.75 % by weight water. Preferably, the hydrated bait contains from 2 to 50 % by weight of a nitrogen source, most preferably 5 to 20 %. Preferably, the hydrated bait contains from 0.25 to 10 % by weight of a superabsorbent polymer, most preferably 1 to 5 %. Preferably, the hydrated bait contains from 0.25 to 20 % by weight of a lipid component, most preferably 1 to 5 %. These amounts are based on weight of the hydrated mollusc bait.

A preferred composition of the invention (hydrated mollusc bait) is:-
10% w/w wheat bran
10% w/w chicken range layers mash,
1.6% w/w sodium polyacrylate cross-linked, grade Z1069, (Stockhausen GmbH)
78.4% w/w water
2000 *P. hermaphrodita* infective juveniles per gram bait.

A preferred composition of the invention (dry mollusc bait) is:-
46 % w/w wheat bran
46 % w/w chicken range layers mash
8 % w/w sodium polyacrylate.

A particularly preferred composition of the invention (hydrated mollusc bait) is:-
8% w/w wheat bran
8% w/w chicken range layers mash,
1.3% w/w sodium polyacrylate cross-linked, grade Z1069, (Stockhausen GmbH)
2.8% w/w sunflower oil
0.4% w/w lecithin
79.5% w/w water
2000 *P. hermaphrodita* infective juveniles per gram bait.

A particularly preferred composition of the invention (dry mollusc bait) is:-
39.1 % w/w wheat bran
39.1 % w/w chicken range layers mash
6.3 % w/w sodium polyacrylate
13.6 % w/w sunflower oil
1.9 % w/w lecithin.

The dry components of the bait are simply mixed together in the required proportions. Before use, water is added in an amount sufficient to hydrate the superabsorbent polymer. The bait composition may be stored as a dry composition or as a hydrated composition. Nematodes may be added to the hydrated bait composition either prior to storage or immediately prior to use. The hydrated mollusc bait/nematode composition may be stored for a period of from 2 hours to 8 weeks, for example from 1 to 28 days, or from 7 to 14 days, and may be stored at a temperature of from about 0 to 10 °C, most preferably from 1 to 3 °C. The bait/nematode composition is then applied to the area to be treated. Any area where molluscs/slugs are present may be treated, such as a garden, plant pots, greenhouse or field. Thus the invention also provides a method of controlling molluscs which comprises applying to an area subject to infestation therewith a molluscicidal composition as described herein.

The skilled person will be able to determine a suitable application rate for the composition, but for example it may be applied at a rate of approximately 2 to 20 grams/pot or plant, preferably about 10 grams/pot or plant, or at a rate of approximately 5 to 20 baits/m², preferably 10 to 15 baits/m², each bait weighing approximately 10 grams.

As well as providing compositions for controlling molluscs as described above, the invention also provides kits for controlling molluscs. Preferably the kit will comprise, as separate components, (i) a mollusc bait and (ii) *Phasmarhabditis* nematodes, wherein the bait comprises a superabsorbent polymer and a selected nitrogen source. The discussion of these components above is equally relevant to the kits of the invention. The bait, when hydrated, provides an environment suitable for reproduction of nematode. It is preferred that the mollusc bait is provided in dry form, such as a powder, or gel form, and is mixed with water prior to use. Alternatively the bait may be provided in a hydrated form. The nematodes are then added to the hydrated bait before application of the resulting bait/nematode composition to the area to be treated. The kit may also contain instructions for use which describe hydration of the mollusc bait, mixing of the kit components and application of the bait/nematode composition.

Thus the invention also provides a method of controlling molluscs which comprises taking a kit as described herein, adding water to the mollusc bait therein, adding the nematodes to the hydrated mollusc bait and applying the resulting bait/nematode composition to an area subject to infestation with molluscs. The hydrated mollusc bait/nematode composition may be applied immediately to the area to be treated or may be stored prior to use. Preferably the composition may be stored for a period of from 2 hours to 8 weeks, for example from 1 to 28 days, or from 7 to 14 days. Preferably the hydrated mollusc bait/nematode composition is stored at a temperature of from about 0 to 10°C, most preferably from 1 to 3°C.

The compositions, kits and methods of the invention may usefully be used to control molluscs which have infested crops or high value plants since the method is a biological control method and no harmful chemicals are used. They are also useful to treat plants which are particularly susceptible to damage by molluscs (e.g. slugs). For example, the compositions, kits and methods of the invention may be used to treat orchids (Cymbidium species, such as Malinga and Evergreen varieties), green asparagus, Brussels sprouts, cabbage and other Brassicae crops, broadacre crops such as wheat, barley, oats, millets, maize, sorghum, oilseeds, pulses and rice, salad vegetables such as Chinese cabbage, radish and lettuce, bedding plants, herbaceous perennial plants such as hostas, and garden ornamentals and vegetables.

The compositions, kits and methods of the invention are useful to control any of the molluscs listed above, and in particular are useful to control slugs such as *Deroceras reticulatum, Arion lusitanicus, Arion distinctus, Arion ater* and *Lehmannia valentiana* and snails such as *Monacha cantiana.* The invention provides an alternative or additional method to use nematodes to more effectively control molluscs, which is particularly relevant for larger species of molluscs, for example, Arion species, since these species spend less time in the soil. For convenience, the invention is described herein mainly in relation to slug control but it is to be understood that it is also applicable to the control of other molluscs that are harmful to plants in the field or greenhouse, or which carry parasites harmful to humans or animals.

### Examples

The following examples are illustrative of the products and methods of making the same falling within the scope of the present invention. They are not to be considered in any way limitative of the invention. Changes and modifications can be made with respect to the invention. That is, the skilled person will recognise many possible variations in these examples covering a wide range of compositions, ingredients, processing methods, and mixtures, and can adjust the naturally occurring levels of the compounds of the invention for a variety of applications.

### Example 1- Composition

The composition used in the following examples is:-
10% w/w wheat bran, Marriages Animal Feeds, Chelmer Mills, Essex, CM1 1PN, UK
10% w/w chicken Range layers mash, HG Chadwell & Sons, Copdock Mill, Ipswich, Suffolk, IP8 3LA, UK
1.6% w/w sodium polyacrylate cross-linked, grade Z1069, Stockhausen GmbH, Bakerpfad 25, 47805 Krefeld, Germany
78.4% w/w water
2000 *P. hermaphrodita* infective juveniles per gram bait, Nemaslug^{RTM}, Becker Underwood, Harwood Industrial Estate, Littlehampton, West Sussex, BN17 7AU, UK

The bait is applied to plant pots where slugs are present at a rate of approximately 10 grams/pot.

### Example 2 - Comparison of nematode numbers on potato and bran baits

Approximately 10 grams of gel/bran bait, with 2000 nematodes/gram were applied to 15 pots of Cymbidium plants. Approximately 6 grams disks of potatoes with 6000 nematodes disk were applied to a further 14 pots of Cymbidium in the glasshouse at the same time. After 90 days in the glasshouse the baits were recovered into plastic petri dishes and assessed for nematode numbers. The results are shown in Table 1 below.

**Table 1: Recovery of nematodes from Bran gel and Potato baits after 90 days in glasshouse**

| | Mean nematodes/gram | Standard deviation |
|---|---|---|
| Bran gel formulaton | 6350 | 7592 |
| Potato disks | 19 | 19 |

The results in Table 1 show that the nematode numbers in the bran gel bait were higher than originally applied. Conversely the number of nematodes remaining on the potato bait was very low. Therefore, in contrast to the potato disks, the bran gel formulation allows the nematodes to grow and reproduce.

### Example 3 - Nematodes numbers versus time in 3 bait formulations

Three nematode baits were prepared as follows :-
(1) Potato disks (Celevita aadappel schijjes, product no. 45) (Potato bait)
(2) Bran - layers mash - polyacrylate (25 grams wheat bran, 25 g layer mash, 4 g polyacrylate, 250 ml water) (bran gel bait)
(3) Polyacrylate gel (10 g polyacrylate + 200 ml water) (Control)

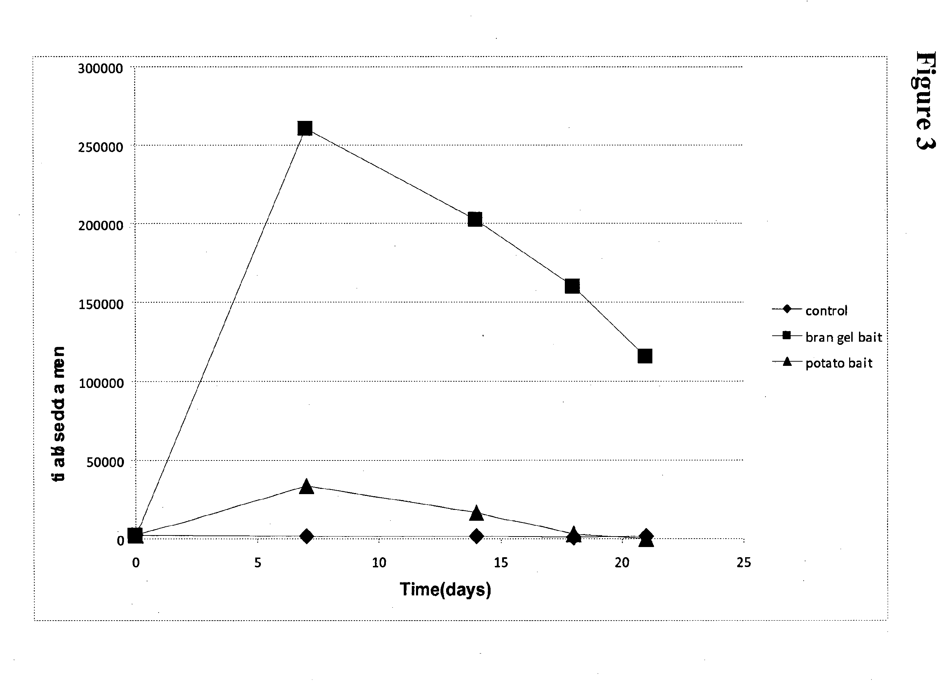

20 samples of each treatment were weighed into petri dishes and each sample inoculated with 2000 *P. hermaphrodita.* The petri dishes were incubated at 15 °C and assessments of total nematode numbers were made at 7, 14, 18 and 21 days. Assessments were carried out in triplicate. The results are shown graphically in Figure 3.

The results indicate that the number of nematodes in the bran gel treatment increased dramatically from an initial number of 2000 per bait to 250,000 nematodes bait at day 7. In contrast, no increase in the number of nematodes was seen in the gel bait (control) and only a small increase was seen in the potato bait.

The number of nematodes in the bran gel treatment remained substantially higher than the other treatments for the duration of the experiment. This demonstrates that the bran gel bait allows the nematodes to increase in numbers and retain these higher levels of viability for longer. The potato bait allowed an initial small increase in numbers which was not sustained, which is due to a lack of water and available nutrients, in contrast to the hydrated bran gel combination.

### Example 4 - Field application of baits to control the slug: Lehmannia valentiana in Cymbidium crops (variety Malinga)

*Cymbidium sp.* are orchids that are grown commercially for their flowers. The crop is however highly susceptible to damage from slugs.

In this example the application of potato and bran gel baits was compared against a control. All the treatments, including the control, also included treatment with metaldehydye (Brabant Slakkendood), iron phosphate (Ferramol) and methiocarb (Mesurol). The variety of Cymbidium in this trial was variety Malinga.

### Treatments

(1) Control- conventional slug pellets alone
(2) Potato slices with 1.18 million *P. hermaphrodita* /kg potato (Nematode SPS, Horticoop)
(3) Bran gel bait as in Example 3

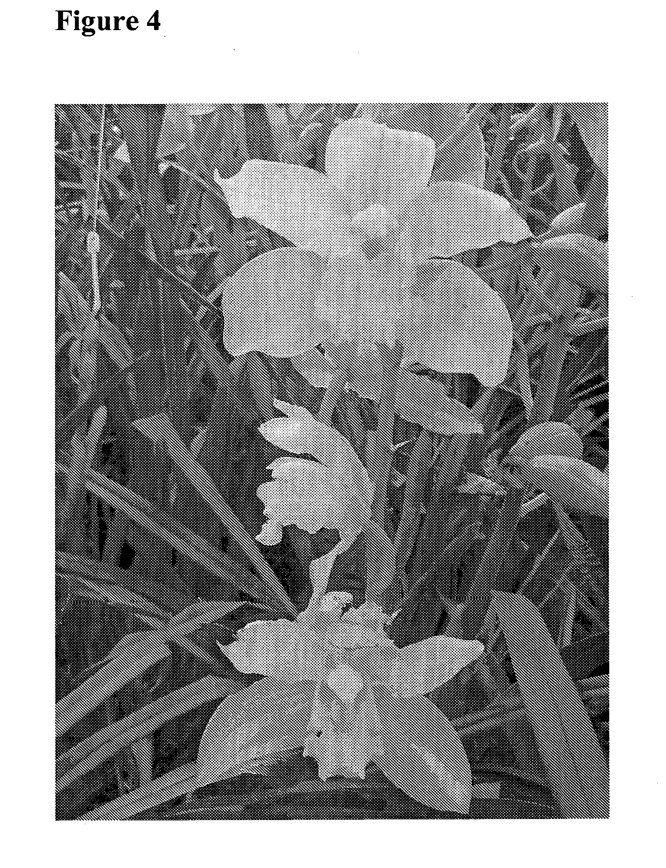

Treatments 2 and 3 were applied on weeks 40 (30/09/09) and 42 (13/10/09). Potato slices were applied at the rate of one slice per pot. The bran bait was applied using a 33 3 mm ice cream scoop giving a rate of 12 grams per pot. The number of stems damaged by slugs was assessed and Figure 4 shows examples of an undamaged Cymbidium stem (top) and a slug-damaged flower (bottom).

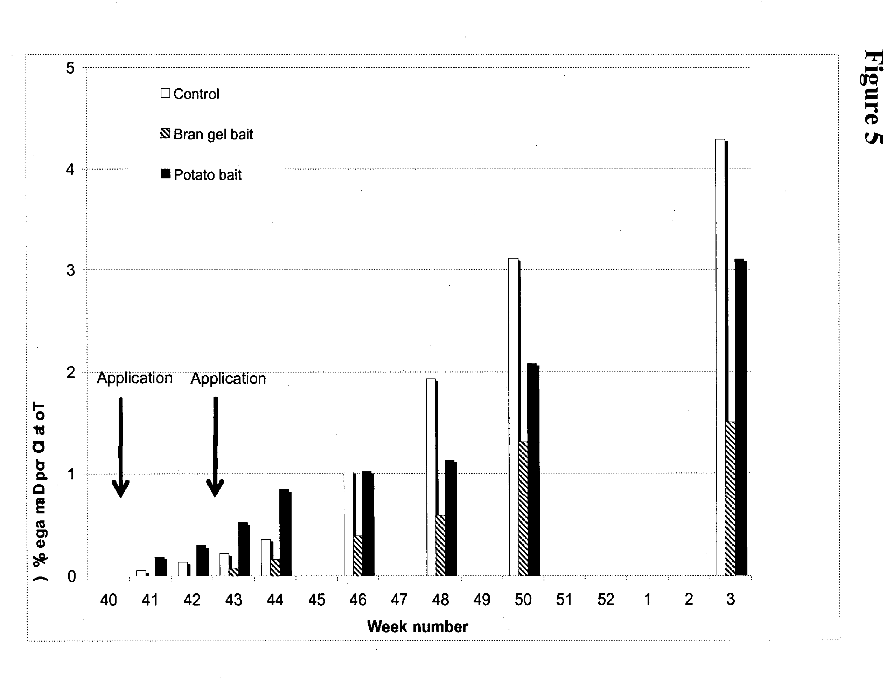

Figure 5 shows the proportion of damaged stems (cumulative damage) versus time. It can be seen that the pots treated with the bran gel bait suffered less damage than the controls and the existing commercially available Nematode SPS potato-based system. In particular, 15 weeks after the initial application the total crop damage was over 4 % with the conventional slug pellets, over 3 % with the Nematode SPS and about 1.5 % with the bran gel bait according to the invention.

### Example 5 - Field application of baits to control the slug: Lehmannia valentiana in Cymbidium crops (variety Evergreen)

This Example was carried out in the same way Example 4, except that the variety of Cymbidium was Evergreen and there was only one application of the baits at week 40 (30/09/09).

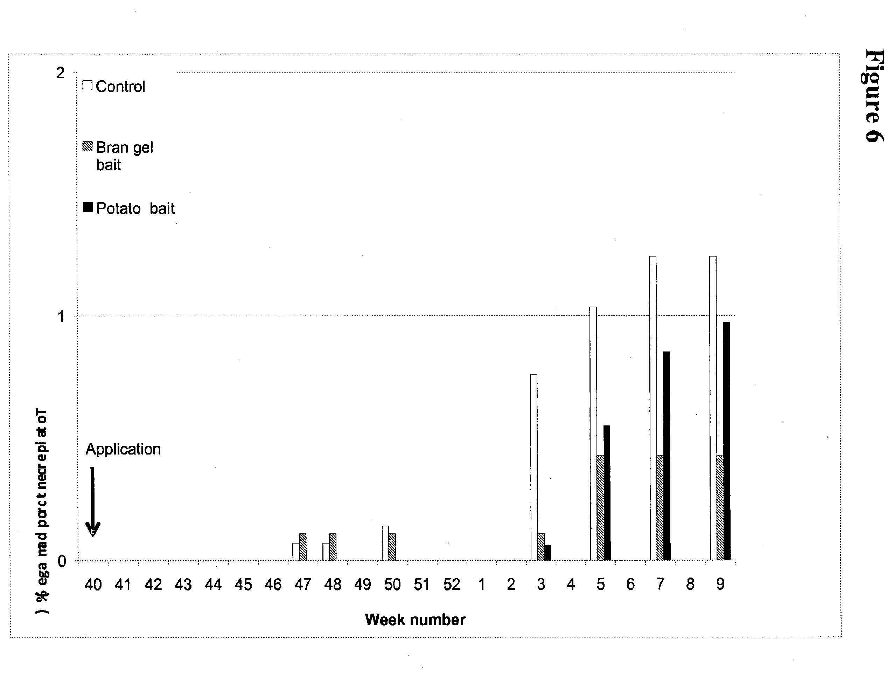

The results are shown in Figure 6, which shows cumulative damage versus time. The results show that the proportion of damaged stems was lower in the bran gel bait treatments of the present invention than in the control and potato bait treatments. Twenty weeks after application of the baits, the proportion of damaged stems in the bran gel bait treatments was less than half compared to both the control and the potato bait treatments.

### Example 6 - Enhancing nematode reproduction by adding additional nutrients

In this example lipids and additional nitrogen were added to the nutrient bait composition described in Example 1 and the effect on nematode reproduction observed.

### Treatments

(1) Control - 10 g wheat bran, 10 g layers mash, 1.6 g sodium polyacrylate, 100 ml water.
(2) Lipid - As (1), but with 100 ml water replaced with 100 ml lipid emulsion comprising 3.5 g sunflower oil, 0.5 g fluid lecithin (Emulfluid^{RTM}, Cargill), made up to 100 ml with water.
(3) Lipid plus nitrogen - As (2), but with the lipid emulsion also containing 1 g yeast extract, 0.5 g kidney extract, 0.5 g powdered egg, 0.5 g soy isolate.

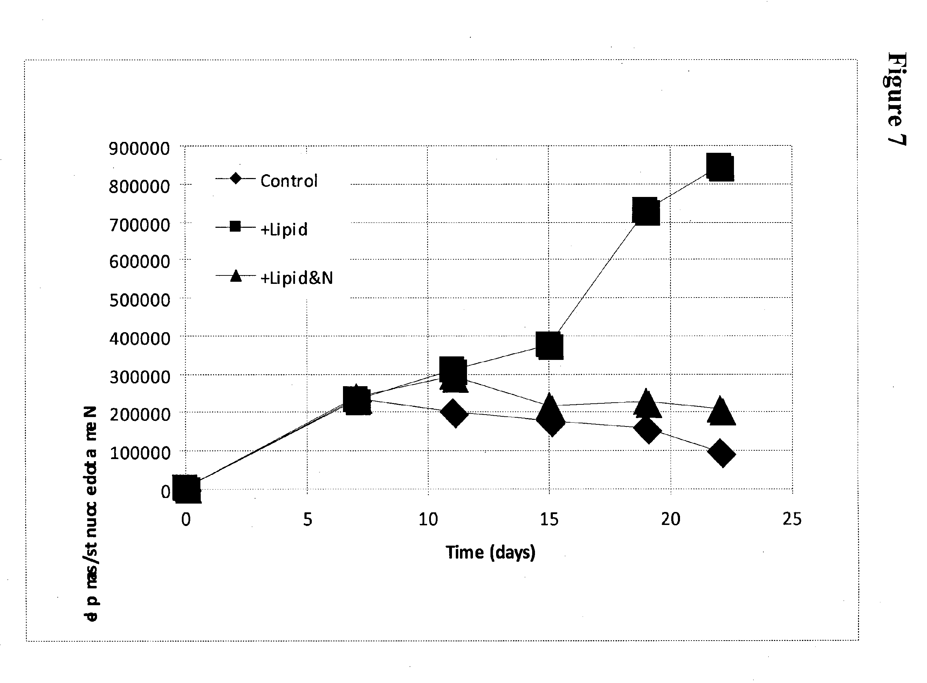

Nematodes were added to each of the treatments at the rate of 2000 nematodes per gram. The resulting treatments was weighed out into 5 gram aliquots into 9 cm petri dishes and incubated at 15 °C. Nematode numbers were assessed at 7, 11, 15, 19 and 22 days. The results for nematode numbers versus time is shown in Figure 7.

It can be seen that the nematode numbers increased substantially over the first 7 days to over 200,000 for all treatments. The treatment supplemented with lipid plus nitrogen (3) caused a further continued increase in nematode numbers, having a moderately beneficial effect. The treatment supplemented with lipids (2) caused a substantial further increase in the number of nematodes to over 800,000 after 22 days. It is expected that this increase in nematodes numbers would improve the performance of the bait in the field.

## Claims

1. A kit for controlling molluscs which comprises (i) a mollusc bait and (ii) *Phasmarhabditis* nematodes, wherein the mollusc bait comprises a superabsorbent polymer and a nitrogen source selected from one or more of brans of rice, corn, wheat, oats and barley; chicken layers mash; whole yeast; yeast extracts; yeast peptones; powdered whole egg; egg yolk; casein; casein peptones; extracts and peptones of meats, fish, dairy products; protein extracts and peptones of plant matter e.g. soya protein, soya isolates and soya peptone; and animal feeds of dairy cows, beef cows, sheep, pigs or poultry.

2. The kit according to claim 1 wherein the bait comprises between 10 to 99 % by weight of the nitrogen source, based on the weight of the dry bait.

3. The kit according to claim 1 or 2 wherein the bait comprises between 1 to 40 % by weight of the superabsorbant polymer, based on the weight of the dry bait.

4. The kit according to any of the preceding claims wherein the bait further comprises water.

5. A composition for controlling molluscs comprising a mollusc bait and a nematode species of the genus *Phasmarhabditis*, wherein the mollusc bait comprises a superabsorbent polymer, a nitrogen source and water, wherein the nitrogen source is selected from one or more of brans of rice, corn, wheat, oats and barley; chicken layers mash; whole yeast; yeast extracts; yeast peptones; powdered whole egg; egg yolk; casein; casein peptones; extracts and peptones of meats, fish, dairy products; protein extracts and peptones of plant matter e.g. soya protein, soya isolates and soya peptone; and animal feeds of dairy cows, beef cows, sheep, pigs or poultry.

6. The composition or kit according to any of the preceding claims wherein the nematode is *Phasmarhabditis neopapillosa* or *Phasmarhabditis hermaphrodita,* preferably *Phasmarhabditis hermaphrodita.*

7. The composition or kit according to any of the preceding claims wherein the nematode is present as dauer larvae.

8. The composition or kit according to any of the preceding claims wherein the superabsorbent polymer is a cross-linked salt of sodium or potassium polyacrylate or polyacrylamide.

9. The composition or kit according to any of the preceding claims wherein the bait further comprises a lipid component, which is preferably an emulsified vegetable lipid component.

10. The composition or kit according to claim 9 wherein the emulsified vegetable lipid component is selected from one or more of vegetable oils such as sunflower, corn, rapeseed and soya bean oil; emulsifiers such as lecithin, egg yolk, monoglycerides, diglycerides, sodium stearoyl acetate and sorbitan fatty acid esters.

11. The composition or kit according to any of claims 4 to 10 wherein the hydrated bait contains from 20 to 97.75 % by weight water.

12. The composition or kit according to any of claims 4 to 11 which comprises from 2 to 50 % by weight of the nitrogen source, based on the weight of the hydrated bait.

13. The composition or kit according to any of claims 4 to 12 which comprises from 0.25 to 10 % by weight of the superabsorbent polymer, based on the weight of the hydrated bait.

14. The composition or kit according to any of claims 9 to 13 which comprises from 0.25 to 20 % by weight of the lipid component, based on the weight of the hydrated bait.

15. The composition or kit according to any of claims 4 to 14 which comprises from 1 to 5000 infective juvenile nematodes per gram of hydrated bait.

16. A method of making a composition for controlling molluscs comprising providing a composition comprising a superabsorbent polymer as defined in claim 1 or 8 and a nitrogen source as defined in claim 1, adding water to the composition to form a hydrated mollusc bait, and adding a nematode species of the genus *Phasmarhabditis* as defined in claim 1 or 6.

17. A method of controlling molluscs which comprises applying to an area subject to infestation therewith a composition according to any of claims 5 to 15 or a composition obtainable by the method of claim 16.

18. A method of controlling molluscs which comprises taking the kit of any of claims 1 to 3 or 6 to 15, adding water to the mollusc bait therein, adding the nematodes to the hydrated mollusc bait and applying the resulting composition to an area subject to infestation with molluscs.

19. A method of controlling molluscs according to claim 17 or 18 wherein the hydrated mollusc bait/nematode composition is stored for a period of 2 hours to 8 weeks prior to its application to an area subject to infestation with molluscs.

## Patentansprüche

1. Kit für die Bekämpfung von Mollusken, das Folgendes umfasst: (i) einen Molluskenköder und (ii) Phasmarhabditis-Nematoden, wobei der Molluskenköder ein Superabsorber-Polymer und eine Stickstoffquelle, ausgewählt aus einem oder mehreren aus Folgendem:
Reiskleie, Maiskleie, Weizenkleie, Haferkleie und Gerstenkleie; Legehennenweichfutter; Vollhefe, Hefeextrakte; Hefepeptone; Volleipulver; Eidotter; Casein; Caseinpeptone; Extrakte und Peptone von Fleisch, Fisch, Milchprodukten; Proteinextrakte und Peptone von pflanzlichem Material, z.B. Sojaprotein, Sojaisolate und Sojapepton; sowie Tierfutter für Milchkühe, Fleischrinder, Schafe, Schweine oder Geflügel umfasst.

2. Kit nach Anspruch 1, wobei der Köder zwischen 10 und 99 Gew.-% der Stickstoffquelle in Bezug auf das Gewicht des trockenen Köders umfasst.

3. Kit nach Anspruch 1 oder 2, wobei der Köder zwischen 1 und 40 Gew.-% des Superabsorber-Polymers in Bezug auf das Gewicht des trockenen Köders umfasst.

4. Kit nach einem der vorhergehenden Ansprüche, wobei der Köder weiterhin Wasser umfasst.

5. Zusammensetzung zum Bekämpfen von Mollusken, umfassend einen Molluskenköder und eine Nematodenart der Gattung *Phasmarhabditis,* wobei der Molluskenköder ein Superabsorber-Polymer, eine Stickstoffquelle und Wasser, wobei die Stickstoffquelle ausgewählt ist aus einem oder mehreren aus Folgendem: Reiskleie, Maiskleie, Weizenkleie, Haferkleie und Gerstenkleie; Legehennenweichfutter; Vollhefe, Hefeextrakte; Hefepeptone; Volleipulver; Eidotter; Casein; Caseinpeptone; Extrakte und Peptone von Fleisch, Fisch, Milchprodukten; Proteinextrakte und Peptone von pflanzlichem Material, z.B. Sojaprotein, Sojaisolate und Sojapepton; sowie Tierfutter für Milchkühe, Fleischrinder, Schafe, Schweine oder Geflügel umfasst.

6. Zusammensetzung oder Kit nach einem der vorhergehenden Ansprüche, wobei es sich bei dem Nematoden um *Phasmarhabditis neopapillosa* oder *Phasmarhabditis hermaphrodita*, vorzugsweise um *Phasmarhabditis hermaphrodita*, handelt.

7. Zusammensetzung oder Kit nach einem der vorhergehenden Ansprüche, wobei der Nematode in Form einer Dauerlarve vorliegt.

8. Zusammensetzung oder Kit nach einem der vorhergehenden Ansprüche, wobei es sich bei dem Superabsorber-Polymer um ein vernetztes Natrium- oder Kaliumpolyacrylat- oder -polyacrylamidsalz handelt.

9. Zusammensetzung oder Kit nach einem der vorhergehenden Ansprüche, wobei der Köder weiterhin eine Lipidkomponente, bei der es sich vorzugsweise um eine emulgierte pflanzliche Lipidkomponente handelt, umfasst.

10. Zusammensetzung oder Kit nach Anspruch 9, wobei die emulgierte pflanzliche Lipidkomponente aus einem oder mehreren der folgenden ausgewählt ist: pflanzliche Öle, wie Sonnenblumenöl, Maiskeimöl, Rapsöl und Sojaöl; Emulgatoren wie Lecithin, Eidotter, Monoglyceride, Diglyceride, Natriumstearoylacetat und Sorbitanfettsäureester.

11. Zusammensetzung oder Kit nach einem der Ansprüche 4 bis 10, wobei der hydratisierte Köder 20 bis 97,75 Gew.-% Wasser enthält.

12. Zusammensetzung oder Kit nach einem der Ansprüche 4 bis 11, die/das 2 bis 50 Gew.-% der Stickstoffquelle in Bezug auf das Gewicht des hydratisierten Köders umfasst.

13. Zusammensetzung oder Kit nach einem der Ansprüche 4 bis 12, die/das 0,25 bis 10 Gew.-% des Superabsorber-Polymers in Bezug auf das Gewicht des hydratisierten Köders umfasst.

14. Zusammensetzung oder Kit nach einem der Ansprüche 9 bis 13, die/das 0,25 bis 20 Gew.-% der Lipidkomponente in Bezug auf das Gewicht des hydratisierten Köders umfasst.

15. Zusammensetzung oder Kit nach einem der Ansprüche 4 bis 14, die/das 1 bis 5000 infektionsfähige juvenile Nematoden pro Gramm hydratisiertem Köder umfasst.

16. Verfahren zur Herstellung einer Zusammensetzung für die Bekämpfung von Mollusken, umfassend das Bereitstellen einer Zusammensetzung, umfassend ein Superabsorber-Polymer wie in Anspruch 1 oder 8 definiert und eine Stickstoffquelle wie in Anspruch 1 definiert, Versetzen der Zusammensetzung mit Wasser unter Bildung eines hydratisierten Molluskenköders und Hinzufügen einer Nematodenart der Gattung *Phasmarhabditis* wie in Anspruch 1 oder 6 definiert.

17. Verfahren zum Bekämpfen von Mollusken, bei dem man auf eine Fläche, die damit verseucht ist, eine Zusammensetzung nach einem der Ansprüche 5 bis 15 oder eine Zusammensetzung, die nach dem Verfahren nach Anspruch 16 erhältlich ist, ausbringt.

18. Verfahren zum Bekämpfen von Mollusken, bei dem man das Kit nach einem der Ansprüche 1 bis 3 oder 6 bis 15 nimmt, den darin befindlichen Molluskenköder mit Wasser versetzt, den hydratisierten Molluskenköder mit den Nematoden versetzt und die erhaltene Zusammensetzung auf eine Fläche ausbringt, die mit Mollusken verseucht ist.

19. Verfahren zum Bekämpfen von Mollusken nach Anspruch 17 oder 18, wobei die hydratisierte Molluskenköder/Nematoden-Zusammensetzung für einen Zeitraum von 2 Stunden bis 8 Wochen vor ihrer Ausbringung auf eine Fläche, die mit Mollusken verseucht ist, aufbewahrt wird.

## Revendications

1. Kit destiné à contrôler des mollusques, comprenant (i) un appât pour mollusques et (ii) des nématodes de type *Phasmarhabditis,* dans lequel l'appât pour mollusques comprend un polymère super-absorbant et une source d'azote choisie parmi un ou plusieurs parmi des sons de riz, de maïs, de blé, d'avoine et d'orge ; du mash pour poules pondeuses ; de la levure complète ; des extraits de levure ; des peptones de levure ; de l'oeuf entier en poudre ; du jaune d'oeuf ; de la caséine ; des peptones de caséine ; des extraits et des peptones de viande, poisson, produits laitiers ; des extraits de protéines et des peptones de matières végétales, par exemple des protéines de soja, des isolats de soja et des peptones de soja ; et des aliments pour animaux de type vaches laitières, bovins à viande, ovins, porcins ou volailles.

2. Kit selon la revendication 1, dans lequel l'appât comprend de 10 à 99% en poids de la source d'azote, sur la base du poids de l'appât sec.

3. Kit selon la revendication 1 ou 2, dans lequel l'appât comprend de 1 à 40% en poids du polymère super-absorbant, sur la base du poids de l'appât sec.

4. Kit selon l'une quelconque des revendications précédentes, dans lequel l'appât comprend en outre de l'eau.

5. Composition destinée à contrôler des mollusques, comprenant un appât pour mollusques et une espèce de nématodes du genre *Phasmarhabditis,* dans laquelle l'appât pour mollusques comprend un polymère super-absorbant, une source d'azote et de l'eau, où la source d'azote est choisie parmi un ou plusieurs parmi des sons de riz, de maïs, de blé, d'avoine et d'orge ; du mash pour poules pondeuses ; de la levure complète ; des extraits de levure ; des peptones de levure ; de l'oeuf entier en poudre ; du jaune d'oeuf ; de la caséine ; des peptones de caséine ; des extraits et des peptones de viande, poisson, produits laitiers ; des extraits de protéines et des peptones de matières végétales, par exemple des protéines de soja, des isolats de soja et des peptones de soja ; et des aliments pour animaux de type vaches laitières, bovins à viande, ovins, porcins ou volailles.

6. Composition ou kit selon l'une quelconque des revendications précédentes, dans laquelle ou lequel le nématode est *Phasmarhabditis neopapillosa* ou *Phasmarhabditis hermaphrodita,* préférablement *Phasmarhabditis hermaphrodita*.

7. Composition ou kit selon l'une quelconque des revendications précédentes, dans laquelle ou lequel le nématode est présent sous forme de larve dauer.

8. Composition ou kit selon l'une quelconque des revendications précédentes, dans laquelle ou lequel le polymère super-absorbant est un sel réticulé de polyacrylate ou de polyacrylamide de sodium ou potassium.

9. Composition ou kit selon l'une quelconque des revendications précédentes, dans laquelle ou lequel l'appât comprend en outre un composant lipidique, qui est de préférence un composant lipidique végétal émulsifié.

10. Composition ou kit selon la revendication 9, dans laquelle ou lequel le composant lipidique végétal émulsifié est choisi parmi un ou plusieurs parmi des huiles végétales telles que l'huile de tournesol, de maïs, de colza ou de soja ; des émulsifiants tels que la lécithine, le jaune d'oeuf, les monoglycérides, les diglycérides, le stéroyl-acétate de sodium et les esters d'acide gras et de sorbitane.

11. Composition ou kit selon l'une quelconque des revendications 4 à 10, dans laquelle ou lequel l'appât hydraté contient de 20 à 97,75% en poids d'eau.

12. Composition ou kit selon l'une quelconque des revendications 4 à 11, comprenant de 2 à 50% en poids de la source d'azote, sur la base du poids de l'appât hydraté.

13. Composition ou kit selon l'une quelconque des revendications 4 à 12, comprenant de 0,25 à 10% en poids du polymère super-absorbant, sur la base du poids de l'appât hydraté.

14. Composition ou kit selon l'une quelconque des revendications 9 à 13, comprenant de 0,25 à 20% en poids du composant lipidique, sur la base du poids de l'appât hydraté.

15. Composition ou kit selon l'une quelconque des revendications 4 à 14, comprenant de 1 à 5000 nématodes juvéniles infectieux par gramme d'appât hydraté.

16. Méthode de préparation d'une composition destinée à contrôler des mollusques, comprenant la fourniture d'une composition comprenant un polymère super-absorbant tel que défini selon la revendication 1 ou 8 et une source d'azote telle que définie selon la revendication 1, l'addition d'eau à la composition pour former un appât pour mollusques hydraté, et l'addition d'une espèce de nématode du genre *Phasmarhabditis* telle que définie selon la revendication 1 ou 6.

17. Méthode de contrôle de mollusques, comprenant l'application à une zone sujette à une infestation par ceux-ci, d'une composition selon l'une quelconque des revendications 5 à 15, ou d'une composition pouvant être obtenue par la méthode selon la revendication 16.

18. Méthode de contrôle de mollusques, comprenant le fait de prendre le kit selon l'une quelconque des revendications 1 à 3 ou 6 à 15, l'addition d'eau à l'appât pour mollusques y étant contenu, l'addition des nématodes à l'appât pour mollusques hydraté et l'application de la composition résultante à une zone sujette à une infestation par des mollusques.

19. Méthode de contrôle de mollusques selon la revendication 17 ou 18, dans laquelle l'appât pour mollusques hydraté/composition de nématodes est stocke(e) pendant une période allant de 2 heures à 8 semaines préalablement à son application à une zone sujette à une infestation par des mollusques.
